(19) Europäisches Patentamt
European Patent Office
Office européen des brevets

(11) EP 4 151 212 A1

(12) **EUROPEAN PATENT APPLICATION**
published in accordance with Art. 153(4) EPC

(43) Date of publication:
**22.03.2023 Bulletin 2023/12**

(21) Application number: **21850938.8**

(22) Date of filing: **29.07.2021**

(51) International Patent Classification (IPC):
***A61K 31/4545*** *(2006.01)* ***A61K 31/138*** *(2006.01)*
***A61K 31/4196*** *(2006.01)* ***A61K 31/565*** *(2006.01)*
***A61K 31/5685*** *(2006.01)* ***A61K 31/58*** *(2006.01)*
***A61K 45/00*** *(2006.01)* ***A61P 15/00*** *(2006.01)*
***A61P 35/00*** *(2006.01)* ***A61P 35/04*** *(2006.01)*
***A61P 43/00*** *(2006.01)*

(52) Cooperative Patent Classification (CPC):
**A61K 31/138; A61K 31/4196; A61K 31/4545; A61K 31/565; A61K 31/5685; A61K 31/58; A61K 45/00; A61P 15/00; A61P 35/00; A61P 35/04; A61P 43/00**

(86) International application number:
**PCT/JP2021/028008**

(87) International publication number:
**WO 2022/025150 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 JP 2020130935**
**29.09.2020 JP 2020163258**

(71) Applicant: **Eisai R&D Management Co., Ltd**
**Bunkyo-ku, Tokyo 112-8088 (JP)**

(72) Inventors:
- **KAWANO Satoshi**
  **Tsukuba-shi, Ibaraki 300-2635 (JP)**
- **MIYANO Saori**
  **Tsukuba-shi, Ibaraki 300-2635 (JP)**
- **NISHIBATA Kyoko**
  **Tsukuba-shi, Ibaraki 300-2635 (JP)**
- **FUKUSHIMA Sayo**
  **Tsukuba-shi, Ibaraki 300-2635 (JP)**

(74) Representative: **Hoffmann Eitle**
**Patent- und Rechtsanwälte PartmbB**
**Arabellastraße 30**
**81925 München (DE)**

(54) **THERAPEUTIC AGENT FOR BREAST CANCER**

(57) Disclosed is a therapeutic agent for breast cancer, said therapeutic agent comprising 5-((2-(4-(1-(2-hydroxyethyl)piperidin-4-yl)benzamide)pyridin-4-yl)oxy)-6-(2-methoxyethoxy)-N-methyl-1H-indole-1-carboxamideor a pharmacologically acceptable salt thereof that is to be administered in combination with an estrogen receptor antagonist or an aromatase inhibitor.

EP 4 151 212 A1

*Fig.1*

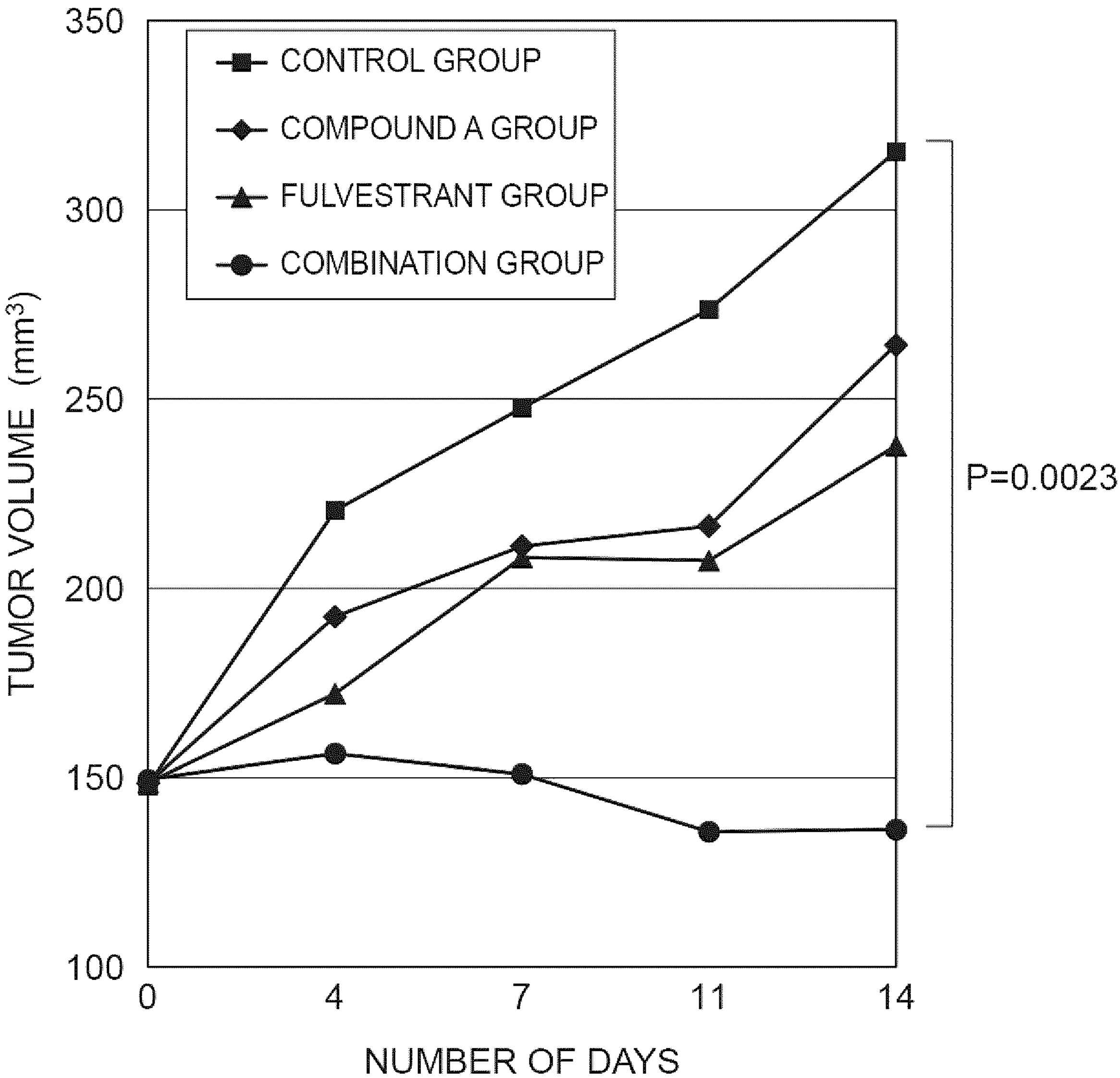

350
300
250
200
150
100
TUMOR VOLUME (mm3)
0
4
7
11
14
NUMBER OF DAYS
CONTROL GROUP
COMPOUND A GROUP
FULVESTRANT GROUP
COMBINATION GROUP
P=0.0023

## Description

## Technical Field

The present invention relates to a therapeutic agent for breast cancer in which a monocyclic pyridine derivative having fibroblast growth factor receptor (FGFR) inhibitory action or a pharmacologically acceptable salt thereof; and an estrogen receptor antagonist or an aromatase inhibitor are used in combination. More specifically, the present invention relates to a therapeutic agent for breast cancer administered in combination with an estrogen receptor antagonist or an aromatase inhibitor, wherein the therapeutic agent for breast cancer comprises 5-((2-(4-(1-(2-hydroxyethyl)piperidin-4-yl)benzamide)pyridin-4-yl)oxy)-6-(2-methoxyethoxy)-N-methyl-1H-indole-1-carboxamide or a pharmacologically acceptable salt thereof.

## Background Art

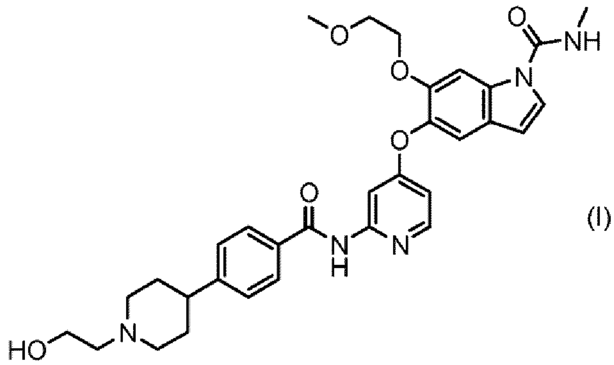

(I)

5-((2-(4-(1-(2-Hydroxyethyl)piperidin-4-yl)benzamide)pyridin-4-yl)oxy)-6-(2-methoxyethoxy)-N-methyl-1H-indole-1-carboxamide represented by Formula (I) is known as an inhibitor against FGFR1, FGFR2, and FGFR3, and it is reported that the above compound has inhibitory action on cell proliferation of stomach cancer, lung cancer, bladder cancer, and endometrial cancer (Patent Literature 1). It is reported that the above compound exerts a high therapeutic effect against bile duct cancer (Patent Literature 2), breast cancer (Patent Literature 3), and hepatocellular carcinoma (Patent Literature 4). As a pharmacologically acceptable salt of the above compound, succinate and maleate are known (Patent Literature 5).

Estrogen receptor antagonists such as tamoxifen and fulvestrant are used for treating estrogen receptor-positive breast cancer (Non Patent Literature 1).

Aromatase inhibitors such as exemestane and anastrozole inhibit aromatase which converts androgen secreted from the adrenal cortex into estrogen (Non Patent Literature 2). For this reason, they are used for treating estrogen receptor-positive breast cancer similarly to the estrogen receptor antagonists. In general, aromatase inhibitors are used in postmenopausal breast cancer patients, whereas estrogen receptor antagonists are used in premenopausal breast cancer patients.

Breast cancer is classified based on the presence or absence of expression of estrogen receptor, progesterone receptor, and human epidermal growth factor receptor type 2 (HER2), and, together with surgical removal of a lesion part, drug therapy is performed depending on the classification.

## Citation List

## Patent Literature

[Patent Literature 1] US Patent Publication No. 2014-0235614
[Patent Literature 2] US Patent Publication No. 2018-0015079
[Patent Literature 3] US Patent Publication No. 2018-0303817
[Patent Literature 4] WO 2019/189241
[Patent Literature 5] US Patent Publication No. 2017-0217935

## Non-Patent Literature

[Non Patent Literature 1] Howell et al., "Comparison of Fulvestrant Versus Tamoxifen for the Treatment of Advanced Breast Cancer in Postmenopausal Women Previously Untreated With Endocrine Therapy: A Multinational, Double-Blind, Randomized Trial", Journal of Clinical Oncology, 2004, 22(9), 1605-1613.

[Non Patent Literature 2] Deeks et al., "Exemestane A Review of its Use in Postmenopausal Women with Breast Cancer" Drugs, 2009, 69(7), 889-918

## Summary of Invention

### Technical Problem

An object of the present invention is to provide a therapeutic agent for breast cancer which involves combinational administration of a plurality of medicines.

### Solution to Problem

In view of such circumstances, the present inventors have conducted extensive studies, and, as a result, they have found that administration of a combination of the above-described compound represented by Formula (I) and an estrogen antagonist or an aromatase inhibitor exerts a high therapeutic effect against breast cancer, thus leading to realization of the present invention.

That is, the present invention provides [1] to [18] below.

[1] A therapeutic agent for breast cancer, comprising: 5-((2-(4-(1-(2-hydroxyethyl)pipendin-4-yl)benzamide)pyridin-4-yl)oxy)-6-(2-methoxyethoxy)-N-methyl-1H-indole-1-carboxamide represented by Formula (I):

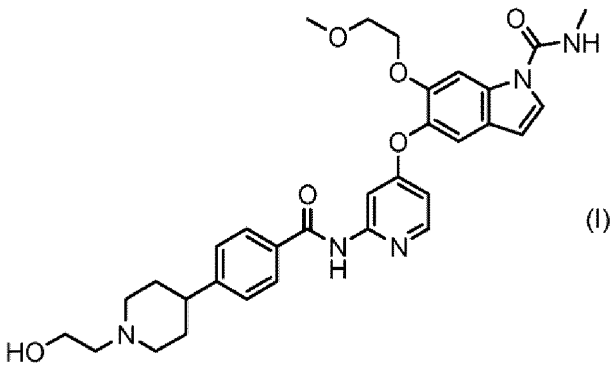

(I)

or a pharmacologically acceptable salt thereof which is administered in combination with an estrogen receptor antagonist or an aromatase inhibitor.

[2] A pharmaceutical composition for treating breast cancer, comprising: 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof which is administered in combination with an estrogen receptor antagonist or an aromatase inhibitor.

[3] A pharmaceutical composition for treating breast cancer, comprising: 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof; and an estrogen receptor antagonist or an aromatase inhibitor.

[4] A kit for treating breast cancer, comprising: a preparation comprising 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof; and a preparation comprising an estrogen receptor antagonist or an aromatase inhibitor.

[5] A method for treating breast cancer, comprising: administering 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof; and an estrogen receptor antagonist or an aromatase inhibitor to a patient in need thereof.

[6] 5-((2-(4-(1-(2-Hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof for use in treatment of breast cancer, which is administered in combination with an estrogen receptor antagonist or an aromatase inhibitor.
[7] A combination for treating breast cancer, comprising: 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof; and an estrogen receptor antagonist or an aromatase inhibitor.
[8] Use of 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof, which is administered in combination with an estrogen receptor antagonist or an aromatase inhibitor, for the manufacture of a therapeutic agent for breast cancer.
[9] The therapeutic agent, composition, kit, method, compound, combination, or use, wherein 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof; and the estrogen receptor antagonist or the aromatase inhibitor are administered simultaneously or separately.
[10] The therapeutic agent, composition, kit, method, compound, combination, or use, wherein the pharmacologically acceptable salt is 1.5 succinate.
[11] The therapeutic agent, composition, kit, method, compound, combination, or use, wherein the estrogen receptor antagonist is fulvestrant, tamoxifen or a pharmacologically acceptable salt thereof, or mepitiostane.
[12] The therapeutic agent, composition, kit, method, compound, combination, or use, wherein the estrogen receptor antagonist is fulvestrant.
[13] The therapeutic agent, composition, kit, method, compound, combination, or use, wherein the aromatase inhibitor is exemestane, anastrozole, or letrozole.
[14] The therapeutic agent, composition, kit, method, compound, combination, or use, wherein the aromatase inhibitor is exemestane.
[14a] The therapeutic agent, composition, kit, method, compound, combination, or use, wherein the aromatase inhibitor is letrozole.
[15] The therapeutic agent, composition, kit, method, compound, combination, or use, wherein the breast cancer is estrogen receptor-positive.
[16] The therapeutic agent, composition, kit, method, compound, combination, or use, wherein the breast cancer is locally advanced breast cancer, metastatic breast cancer, recurrent breast cancer, or unresectable breast cancer.
[17] The therapeutic agent, composition, kit, method, compound, combination, or use, which is for use in treatment of breast cancer fibroblast growth factor receptor (FGFR).
[18] The therapeutic agent, composition, kit, method, compound, combination, or use, wherein FGFR is FGFR1, FGFR2, or FGFR3.

## Advantageous Effects of Invention

By administering a combination of the compound represented by Formula (I) and an estrogen antagonist or an aromatase inhibitor, there is a probability that an effect of reducing a tumor volume against breast cancer may be exhibited.

## Brief Description of Drawings

Fig. 1 is a graph showing changes in average tumor volume in each group after starting drug administration in Example 1.
Fig. 2 is a graph showing changes in average tumor volume in each group after starting drug administration in Example 4.

## Description of Embodiments

A compound represented by Formula (I) or a pharmacologically acceptable salt thereof according to the present invention can be produced through a method disclosed in Patent Literature 1.
In the present specification, examples of pharmacologically acceptable salts include a salt with an inorganic acid, a salt with an organic acid, and a salt with an acidic amino acid.
Examples of suitable examples of salts with inorganic acids include salts with hydrochloric acid, hydrobromic acid, sulfuric acid, nitric acid, and phosphoric acid.
Suitable examples of salts with organic acids include salts with acetic acid, succinic acid, fumaric acid, maleic

acid, tartaric acid, citric acid, lactic acid, stearic acid, benzoic acid, methanesulfonic acid, ethanesulfonic acid, and p-toluenesulfonic acid.

Examples of suitable examples of salts with acid amino acids include salts with aspartic acid and glutamic acid.

A preferred pharmacologically acceptable salt is succinate or maleate, and a more preferred salt is succinate. 1.5 Succinate is particularly preferable (hereinafter, 1.5 succinate of the compound represented by Formula (I) is denoted as a compound A).

The therapeutic agent for breast cancer according to the present invention can be administered orally in a form of a solid preparation such as a tablet, granules, fine granules, powders, or a capsule, a liquid, a jelly, a syrup, or the like. In addition, the therapeutic agent for breast cancer according to the present invention may be administered parenterally in a form of an injection, a suppository, an ointment, a poultice, or the like.

The therapeutic agent for breast cancer according to the present invention can be formulated by the methods described in the Japanese Pharmacopoeia (JP), the European Pharmacopoeia (EP), or the United States Pharmacopeia (USP).

The dose of the compound represented by Formula (I) or a pharmacologically acceptable salt thereof can be appropriately selected depending on the severity of symptoms, the age, sex, body weight, and differential sensitivity of a patient, the administration method, the administration period, the administration intervals, the dosage form, and the like. In a case of oral administration to an adult (body weight of 60 kg), the dose is 0.5 mg to 5 g, preferably 1 mg to 1 g, and furthermore preferably 1 mg to 500 mg per day. This dose can be administered in 1 to 3 divided portions per day.

In the present specification, the estrogen receptor antagonist means a medicine that binds to an estrogen receptor expressed in breast cancer cells. By the mechanism, the estrogen receptor antagonist can inhibit the binding of estrogen receptors to estrogen and suppress the proliferation of breast cancer cells.

Suitable examples of estrogen receptor antagonists include fulvestrant, tamoxifen or a pharmacologically acceptable salt thereof (such as citrate), and mepitiostane. Fulvestrant is preferable.

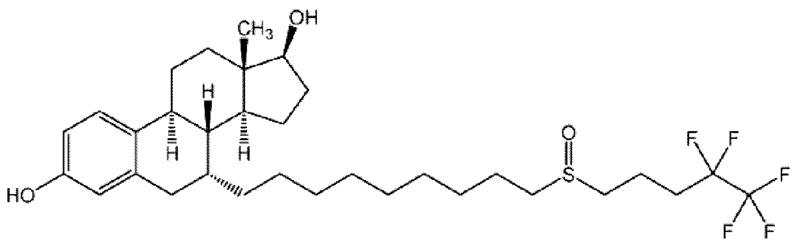

Fulvestrant

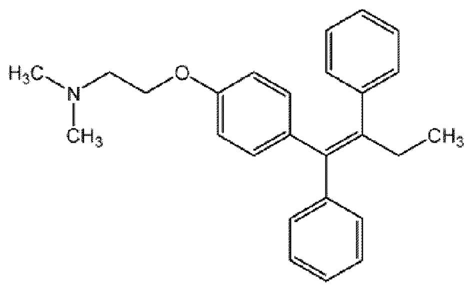

Tamoxifen

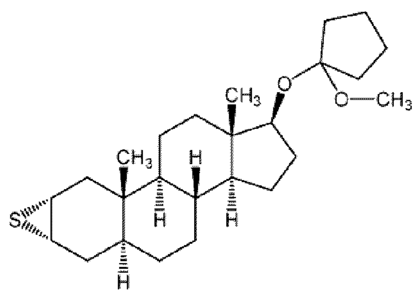

Mepitiostane

In addition, the estrogen receptor antagonist can be elacestrant, H3B-6545, toremifene citrate, SAR439859, AZD9833, rintodestrant, ZN-c5, LSZ102, D-0502, LY3484356, SHR9549, brilanestrant, and giredestrant.

Regarding the dose and administration method of the estrogen receptor antagonist, in a case where, for example, the estrogen receptor antagonist is fulvestrant, 500 mg thereof is administered intramuscularly per dose at an initial dose, 2 weeks later, 4 weeks later, and every 4 weeks thereafter.

In a case where the estrogen receptor antagonist is tamoxifen or a pharmacologically acceptable salt thereof, 20 to 40 mg (as tamoxifen) per day is administered orally in 1 to 2 divided doses.

In a case where the estrogen receptor antagonist is mepitiostane, 20 mg per day is administered orally in 2 divided doses.

In addition, an aromatase inhibitor that inhibits synthesis of estrogen may be used instead of the estrogen receptor antagonist. Suitable examples of aromatase inhibitors include exemestane, anastrozole, and letrozole. Exemestane is preferable.

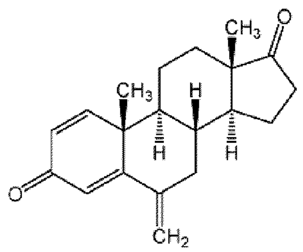

Exemestane

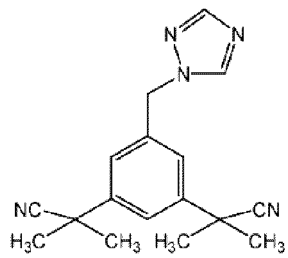

Anastrozole

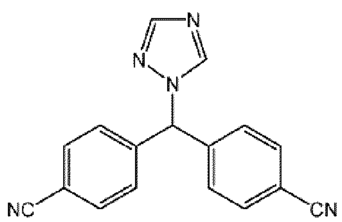

Letrozole

Regarding the dose and administration route of the aromatase inhibitor, in a case where, for example, the aromatase inhibitor is exemestane, 25 mg thereof is administered orally once a day after meals.

In a case where the aromatase inhibitor is anastrozole, 1 mg thereof is administered orally once a day.

In a case where the aromatase inhibitor is letrozole, 2.5 mg thereof is administered orally once a day.

In the present specification, administrating in combination means that a preparation comprising the compound represented by Formula (I) or a pharmacologically acceptable salt thereof; and a preparation comprising an estrogen receptor antagonist or an aromatase inhibitor are administered to a patient simultaneously or separately. In addition, a composition comprising the compound represented by Formula (I) or a pharmacologically acceptable salt thereof; and an estrogen receptor antagonist or an aromatase inhibitor in one preparation may be administered.

In the present specification, breast cancer means a benign or malignant tumor developed in mammary glands (milk ducts and lobules). The breast cancer includes locally advanced breast cancer, metastatic breast cancer, recurrent breast cancer, or unresectable breast cancer.

## Examples

The present invention will be described in more detail with reference to the following examples.

Example 1 Proliferation inhibitory action against human breast cancer patient-derived tumor (OD-BRE-0438) due to combined use of compound A and fulvestrant (trade name: Faslodex intramuscular injection 250 mg, AstraZeneca PLC)

Five NOD-SCID mice (NOD. CB17-Prkdcscid/J, female, Charles River Laboratories Japan, Inc.) were used in each group to evaluate antitumor effect in a case where the compound A and the fulvestrant were administered.

OD-BRE-0438 is a hormone receptor-positive breast cancer patient-derived tumor established by Oncodesign SA. Subculture was performed by subcutaneously transplanting tumor pieces into the NOD-SCID mice. Each of the above-described tumors excised from the mice was chopped into about 5 mm squares, transplanted into the subcutaneous right side of each mouse using a trocar (Φ3.5 mm), and provided for evaluating the antitumor effect.

β-Estradiol (FUJIFILM Wako Pure Chemical Corporation) was made into a solution with 99.5% ethanol (FUJIFILM Wako Pure Chemical Corporation) at a concentration of 1 mg/mL, and then, the solution was prepared to a final concentration of 2.5 μg/mL using sterile water for water supply. This solution was administered to the mice in their drinking water from the day of tumor transplantation to the test end date.

The major axis and minor axis of each tumor was measured with an electronic digital caliper (Digimatic™ Caliper, Mitutoyo Corporation). The volume of each tumor was calculated according to the following equation.

$$\text{Tumor volume (mm}^3\text{)} = \text{longest diameter (mm)} \times \text{short axis (mm)} \times \text{short axis (mm)} / 2$$

The compound A was dissolved in purified water to a concentration of 2.5 mg/mL. As fulvestrant, a commercially available preparation (50 mg/mL) was used as it is.

30 days after tumor transplantation, the mice were assigned to each group so that the average tumor volume was the same.

The start day of administration was set to day 0, and the drug was administered under the following conditions.

The compound A was administered orally to the mice in each group at a dose of 25 mg/kg (10 mL/kg) once a day for 14 consecutive days. In addition, the fulvestrant was injected subcutaneously at a dose of 250 mg/kg (5 mL/kg) on days 0 and 7. A control group was left untreated.

The tumor volume of each of the mice was measured on days 0, 4, 7, 11, and 14. The results (average values) are shown in Table 1 and Fig. 1. Dunnett's multiple test was performed on the tumor volume of the control group and each of the administration groups on day 14.

[Table 1]

| Changes in average tumor volume after starting drug administration ($mm^3$) | | | | | |
|---|---|---|---|---|---|
| | Day 0 | Day 4 | Day 7 | Day 11 | Day 14 |
| Control group ($mm^3$) | 147.9 | 220.7 | 247.6 | 273.7 | 315.5 |
| Compound A 25 mg/kg group ($mm^3$) | 148.6 | 192.6 | 211.2 | 216.5 | 264.3 |
| Fulvestrant 250 mg/kg group ($mm^3$) | 148.7 | 172.2 | 208.2 | 207.4 | 237.6 |
| Combination group ($mm^3$) | 149.6 | 156.4 | 151.0 | 135.8 | 136.4 |

Reference Example 1 Construction of human CYP19A1 expression vector

A PB-CMV-MCS-EF1α-Puro vector (System Bioscience, LLC) was cleaved with Xba I and Not I, and a multiple cloning site was inserted thereinto to prepare a PB510B2 vector. Next, the PB510B2 vector was cleaved with Xba I and Cla I, and ORF of human CYP19A1 (NM_001347249.2) was inserted thereinto to obtain a PB510B2_hCYP19A1 vector.

Reference Example 2 Construction of pC3-PBase vector

A pC3-vector which was a vector obtained by removing a neomycin expression module (SV40 promoter-Neomycin ORF-SV40 poly A) of a pcDNA3 1(-) mammalian expression vector (Thermo Fisher Scientific Inc.) was

constructed. Next, ORF of Super PiggyBac Transposase of Super PiggyBac Transposase Expression Vector (System Bioscience, LLC) was inserted into the downstream of CMV promoter of pC3-vector to obtain pC3-PBase vector.

Reference Example 3 Establishment of human-derived breast cancer cell line ZR-75-1-hCYP19A1

A human-derived breast cancer cell line ZR-75-1 (ECACC) was seeded in a 6-well microplate (FALCON). RPMI-1640 medium (containing 4,500 mg/L glucose, L-glutamine, phenol red, HEPES, and sodium pyruvate, FUJIFILM Wako Pure Chemical Corporation) containing 10% FBS (SIGMA) and penicillin/streptomycin (FUJIFILM Wako Pure Chemical Corporation) was used as a medium. The seeded cells were cultured for 15 days under the conditions of 5% $CO_2$ and 37°C using an incubator.

A liquid A which was obtained by mixing Lipofectamine™ 3000 reagent (Thermo Fisher Scientific Inc.) (3.75 μL) with Opti-MEM™ (Thermo Fisher Scientific Inc.) (125 μL) was prepared. In addition, the above prepared PB510B2_hCYP19A1 vector (2 μg), pC3-PBase (0.5 μg), P3000 reagent (Thermo Fisher Scientific Inc.) (5 μL), and Opti-MEM™ (125 μL) were mixed with each other to prepare a liquid B. These liquids A and B were mixed with each other, allowed to stand at room temperature for 5 minutes, and then added to the above-described ZR-75-1 cells obtained by culture, and the cells were cultured overnight under the conditions of 5% $CO_2$ and 37°C. Thereafter, culture was performed in a medium to which 1 μg/mL puromycin was added.

Reference Example 4 Establishment of human-derived breast cancer cell line MCF-7-hCYP19A1

A human-derived breast cancer cell line MCF-7 (ECACC) was seeded in a 6-well microplate (FALCON). 10% FBS (SIGMA), penicillin/streptomycin (FUJIFILM Wako Pure Chemical Corporation), andE-MEM medium (containing L-glutamine, phenol red, sodium pyruvate, non-essential amino acids, and 1500 mg/L sodium hydrogen carbonate, FUJIFILM Wako Pure Chemical Corporation) were used as a medium. The seeded cells were cultured overnight under the conditions of 5% CO2 and 37°C using an incubator.

A liquid A obtained by mixing Lipofectamine™ 3000 reagent (3.75 μL) with Opti-MEM™ (125 μL) was prepared. In addition, the above prepared PB510B2_hCYP19A1 vector (2 μg), pC3-PBase (0.5 μg), P3000 reagent (5 μL), and Opti-MEM™ (125 μL) were mixed with each other to prepare a liquid B. These liquids A and B were mixed with each other, allowed to stand at room temperature for 5 minutes, and then added to the above-described MCF-7 cells obtained by culture and the cells were cultured for 3 days under the conditions of 5% $CO_2$ and 37°C. Thereafter, the cells were cultured in a medium to which 1 μg/mL puromycin was added for 7 days, and then cultured in a medium to which 1.5 μg/mL puromycin was added.

Example 2 Proliferation inhibitory action against subcutaneously transplanted tumor of human-derived breast cancer cell line ZR-75-1-hCYP19A1 due to combined use of compound A and exemestane

Five BALB/c nude mice (CAnN.Cg-Foxn1/CrlCrlj, female, Charles River Laboratories Japan, Inc.) were used in each group to evaluate an antitumor effect in a case where the compound A and exemestane were administered.

The mice were subcutaneously injected with a mixed anesthetic solution of 0.3 mg/kg medetomidine hydrochloride, 4 mg/kg midazolam, and 5 mg/kg butorphanol tartrate. The skin and peritoneum on the back of each of the mice were incised, the left and right ovaries were resected, and the incision was sutured. Thereafter, 65 mg/kg (13.0 mg/mL, 100 μL/mouse) ampicillin sodium injection (Meiji Seika Pharma Co., Ltd.), 1 mg/kg (0.2 mg/mL, 100 μL/mouse) Antisedan (Nippon Zenyaku Kogyo Co., Ltd.), and 5 mg/kg (1.0 mg/mL, 100 μL/animal) carprofen injection (Zoetis Japan) were administered subcutaneously.

7 days later, androstenedione (Sigma-Aldrich) was administered orally to the mice at a dose of 0.1 mg/mouse (200 μL/mouse) until the test end date.

One day after the start of administration of androstenedione, $1\times10^7$ cells/mouse of a human-derived breast cancer cell line ZR-75-1-hCYP19A1 was transplanted subcutaneously into the right sides of the mice. 20 days after the transplantation of the breast cancer cell line, the mice were assigned to each group so that the average tumor volume was the same.

The compound A was dissolved in purified water to a concentration of 2.5 mg/mL. In addition, Tween (registered trademark) 80 was mixed with a 0.5% methyl cellulose solution to a concentration of 0.4%. Exemestane was dissolved in this mixed solution to a concentration of 5 mg/mL.

The start day of administration was set to day 0, and the drug was administered under the following conditions. The compound A at a dose of 50 mg/kg (20 mL/kg), exemestane at a dose of 1 mg/mouse (200 μL/mouse), or the two agents of the compound A and exemestane (each at the same dose as described above), were administered orally to the mice in each group once a day for 11 consecutive days. A control group was left untreated.

The tumor volume of each of the mice was measured on days 0, 4, 7, and 11. The results (average values) are shown in Table 2. The major axis and minor axis of each tumor was measured with an electronic digital caliper (Digimatic™ Caliper, Mitutoyo Corporation). The volume of each tumor was calculated according to the following equation.

$$\text{Tumor volume (mm}^3\text{) = longest diameter (mm)} \times \text{short axis (mm)} \times \text{short axis (mm) / 2}$$

[Table 2]

| Changes in average tumor volume after starting drug administration ($mm^3$) | | | | |
|---|---|---|---|---|
| | Day 0 | Day 4 | Day 7 | Day 11 |
| Control group ($mm^3$) | 145.4 | 206.8 | 261.8 | 362.9 |
| Compound A 50 mg/kg group ($mm^3$) | 147.3 | 187.7 | 232.8 | 319.9 |
| Exemestane 1 mg/mouse group ($mm^3$) | 146.0 | 184.5 | 235.0 | 329.0 |
| Combination group ($mm^3$) | 145.3 | 184.5 | 218.5 | 277.4 |

Example 3 Effect of combining compound A and letrozole, anastrozole, or exemestane on human-derived breast cancer cell line MCF-7-hCYP19A1 in which human aromatase was expressed

The human breast cancer cell line MCF-7-hCYP19A1 prepared in Reference Example 4 was cultured and maintained in a 5% $CO_2$ incubator using E-MEM medium (containing L-glutamine, phenol red, sodium pyruvate, non-essential amino acids, and 1500 mg/L sodium hydrogen carbonate, FUJIFILM Wako Pure Chemical Corporation) containing 10% FBS (SIGMA), penicillin/streptomycin (FUJIFILM Wako Pure Chemical Corporation), and 1.5 $\mu$g/mL puromycin (hereinafter referred to as a culture medium).

200 $\mu$L of each cell suspension prepared to $0.25 \times 10^4$ cells/mL in the culture medium containing 10% FBS was added to each well of a cell culture plate and cultured overnight in the incubator.

On the next day, the medium was removed, and 100 $\mu$L of phenol red-free RPMI-1640 medium (FUJIFILM Wako Pure Chemical Corporation) containing 10% FBS (charcoal-stripped FBS, GIBCO), penicillin/streptomycin, and 1.5 $\mu$g/mL puromycin (hereinafter referred to as an assay medium) was added thereto, and culture was performed in the incubator.

On the next day, the medium was removed, 100 $\mu$L of an assay medium was added thereto to replace the medium, and the cells were cultured in the incubator for 2 days. Thereafter, the medium was removed, 25 $\mu$L of androstenedione prepared to a final concentration of 10 nmol/L in the assay medium and 25 $\mu$L of each of FGF2 (GIBCO) and FGF10 (R&D systems) respectively prepared to final concentrations of 25 ng/mL and 100 ng/mL in the assay medium were added thereto, and then, 25 $\mu$L of each solution described below was added thereto, and culture was performed in the incubator for 7 days. The medium was removed 3 days and 5 days after addition of the drugs, and 25 $\mu$L of each of the androstenedione, FGF2, and FGF10 solutions and each solution described below was added thereto to replace the medium.

(1) Assay medium containing letrozole (SIGMA), anastrozole (SIGMA), exemestane (SIGMA), or DMSO respectively prepared to final concentrations of 2,000 nmol/L, 10,000 or 2,000 nmol/L, 2,000 nmol/L or 400 nmol/L, and 0.05% in assay medium (used for control group or single agent of Compound A)

(2) Assay medium containing Compound A and DMSO respectively prepared to final concentrations of 1,000 nmol/L and 0.005% in assay medium (used for control group or single drug described in (1))

Using CellTiter Glo™ 2.0 (Promega), the number of cells in each of the wells after culture was calculated based on the amount of ATP contained in the cells obtained by measuring the emission intensity.

After removing the medium from the plate, 50 $\mu$L of a solution obtained by mixing the measurement reagent with the culture medium at 1:1 was added to each well. After mixing the plate in a plate mixer for 5 minutes, the emission was measured using a plate reader (EnVision™, PerkinElmer, Inc.). Average values (n=3) of each group for values obtained by subtracting emission values in cell-free wells from emission values of the wells are shown in Tables 3 to 7.

[Table 3]

| | Emission value (count per second) |
|---|---|
| Control group | 1,481,915 |
| Compound A 1,000 nmol/L | 1,242,509 |

(continued)

| | Emission value (count per second) |
|---|---|
| Letrozole 2,000 nmol/L | 1,410,920 |
| Combined use of Compound A and letrozole | 1,159,491 |

[Table 4]

| | Emission value (count per second) |
|---|---|
| Control group | 1,334,640 |
| Compound A 1,000 nmol/L | 1,157,051 |
| Anastrozole 10,000 nmol/L | 1,443,072 |
| Combined use of Compound A and anastrozole | 924,395 |

[Table 5]

| | Emission value (count per second) |
|---|---|
| Control group | 1,334,640 |
| Compound A 1,000 nmol/L | 1,157,051 |
| Anastrozole 2,000 nmol/L | 1,357,941 |
| Combined use of Compound A and anastrozole | 1,018,781 |

[Table 6]

| | Emission value (count per second) |
|---|---|
| Control group | 1,243,964 |
| Compound A 1,000 nmol/L | 1,164,308 |
| Exemestane 2,000 nmol/L | 1,244,423 |
| Combined use of Compound A and exemestane | 949,300 |

[Table 7]

| | Emission value (count per second) |
|---|---|
| Control group | 1,243,964 |
| Compound A 1,000 nmol/L | 1,164,308 |
| Exemestane 400 nmol/L | 1,324,919 |
| Combined use of Compound A and exemestane | 1,003,903 |

Example 4 Proliferation inhibitory action against subcutaneously transplanted tumor of human-derived breast cancer cell line MCF-7-hCYP19A1 due to combined use of compound A and letrozole

Five BALB/c nude mice (CAnN.Cg-Foxn1/CrlCrlj, female, Charles River Laboratories Japan, Inc.) were used in each group to evaluate an antitumor effect in a case where the compound A and the letrozole were administered.

The mice were subcutaneously injected with a mixed anesthetic solution of 0.3 mg/kg medetomidine hydrochloride, 4 mg/kg midazolam, and 5 mg/kg butorphanol tartrate. The skin and peritoneum on the back of each of the mice were incised, the left and right ovaries were resected, and the incision was sutured. Thereafter, Baytril 2.5% injection (Bayer) diluted with physiological saline (Otsuka Pharmaceutical Factory, Inc.) to a final concentration of 1 mg/mL as

enrofloxacin; and antisedan (Nippon Zenyaku Kogyo Co., Ltd.) diluted with physiological saline (Otsuka Pharmaceutical Factory, Inc.) to a final concentration of 0.2 mg/mL as atipamezole hydrochloride were administered subcutaneously at respective amounts equivalent to 5 mg/kg (100 μL/mouse) and 1 mg/kg (100 μL/mouse).

7 days later, a solution obtained by diluting testosterone enanthate intramuscular injection (Fuji Pharma Co., Ltd.) with sesame oil to a concentration of 15.6 mg/mL was administered intramuscularly at a concentration of 0.1 mL/mouse (1.56 mg/mouse). Thereafter, the solution was administered intramuscularly to the mice once a week until the test end date.

One day after the start of administration of testosterone enanthate, $1\times10^7$ cells/mouse of the human-derived breast cancer cell line MCF-7-hCYP19A1 was transplanted subcutaneously into the right sides of the mice. 9 days after transplantation of the breast cancer cell line, the mice were assigned to each group so that the average tumor volume was the same.

The compound A was dissolved in purified water to a concentration of 2.5 mg/mL. In addition, letrozole was dissolved in sterilized 0.3% hydroxypropyl cellulose water to a concentration of 0.05 mg/mL.

The start day of administration was set to day 0, and the drug was administered under the following conditions. The compound A at a dose of 25 mg/kg (10 mL/kg), letrozole at a dose of 0.01 mg/mouse (200 μL/mouse), or the two agents of the compound A and the letrozole (each at the same dose as described above), were administered orally to the mice in each group once a day for 14 consecutive days. A control group was left untreated.

The tumor volume of each of the mice was measured on days 0, 4, 7, 11, and 14. The results (average values) are shown in Table 8 and Fig. 2. The major axis and minor axis of each tumor was measured with an electronic digital caliper (Digimatic™ Caliper, Mitutoyo Corporation). The volume of each tumor was calculated according to the following equation.

$$\text{Tumor volume (mm}^3\text{)} = \text{longest diameter (mm)} \times \text{short axis (mm)} \times \text{short axis (mm)} / 2$$

Dunnett's multiple test was performed on the tumor volume of the control group and each of the administration groups on day 14.

[Table 8]

| Changes in average tumor volume after starting drug administration ($mm^3$) | | | | | |
|---|---|---|---|---|---|
| | Day 0 | Day 4 | Day 7 | Day 11 | Day 14 |
| Control group ($mm^3$) | 131.7 | 193.9 | 227.8 | 235.9 | 265.2 |
| Compound A 25mg/kg group ($mm^3$) | 130.7 | 155.5 | 182.4 | 224.4 | 267.5 |
| Letrozole 0.01mg/mouse group ($mm^3$) | 132.4 | 147.3 | 140.6 | 159.6 | 162.0 |
| Combination group ($mm^3$) | 132.0 | 117.9 | 118.6 | 132.9 | 137.3 |

**Claims**

1. A therapeutic agent for breast cancer, comprising:
5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I):

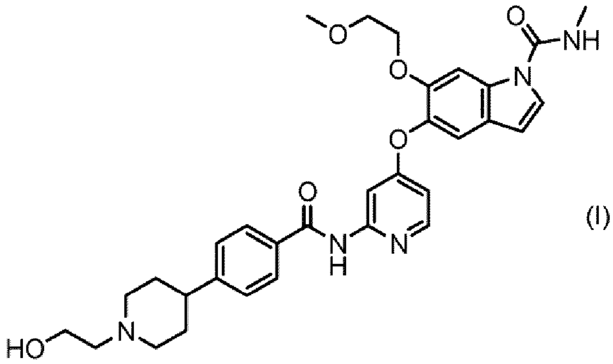

(I)

or a pharmacologically acceptable salt thereof which is administered in combination with an estrogen receptor antagonist or an aromatase inhibitor.

**2.** The therapeutic agent according to claim 1, wherein 5-((2-(4-(1-(2-hydroxyethyl)piperidin-4-yl)benzamide)pyridin-4-yl)oxy)-6-(2-methoxyethoxy)-N-methyl-1H-indole-1-carboxamide represented by Formula (I) or a pharmacologically acceptable salt thereof; and the estrogen receptor antagonist or the aromatase inhibitor are administered simultaneously or separately.

**3.** The therapeutic agent according to claim 1 or 2, wherein the pharmacologically acceptable salt is 1.5 succinate.

**4.** The therapeutic agent according to any one of claims 1 to 3, wherein the estrogen receptor antagonist is fulvestrant, tamoxifen or a pharmacologically acceptable salt thereof, or mepitiostane.

**5.** The therapeutic agent according to any one of claims 1 to 3, wherein the estrogen receptor antagonist is fulvestrant.

**6.** The therapeutic agent according to any one of claims 1 to 3, wherein the aromatase inhibitor is exemestane, anastrozole, or letrozole.

**7.** The therapeutic agent according to any one of claims 1 to 3, wherein the aromatase inhibitor is exemestane.

**8.** The therapeutic agent according to any one of claims 1 to 3, wherein the aromatase inhibitor is letrozole.

**9.** The therapeutic agent according to any one of claims 1 to 8, wherein the breast cancer is estrogen receptor-positive.

**10.** The therapeutic agent according to any one of claims 1 to 9, wherein the breast cancer is locally advanced breast cancer, metastatic breast cancer, recurrent breast cancer, or unresectable breast cancer.

**11.** The therapeutic agent according to any one of claims 1 to 10, which is for use in treatment of breast cancer fibroblast growth factor receptor (FGFR).

**12.** The therapeutic agent according to claim 11, wherein FGFR is FGFR1, FGFR2, or FGFR3.

**13.** A pharmaceutical composition for treating breast cancer, comprising: 5-((2-(4-(1-(2-hydroxyethyl)piperidin-4-yl)benzamide)pyridin-4-yl)oxy)-6-(2-methoxyethoxy)-N-methyl-1H-indole-1-carboxamide or a pharmacologically acceptable salt thereof which is administered in combination with an estrogen receptor antagonist or an aromatase inhibitor.

**14.** A pharmaceutical composition for treating breast cancer, comprising:

5-((2-(4-(1-(2-hydroxyethyl)piperidin-4-yl)benzamide)pyridin-4-yl)oxy)-6-(2-methoxyethoxy)-N-methyl-1H-indole-1-carboxamide or a pharmacologically acceptable salt thereof; and
an estrogen receptor antagonist or an aromatase inhibitor.

**15.** A kit for treating breast cancer, comprising:

a preparation comprising 5-((2-(4-(1-(2-hydroxyethyl)piperidin-4-yl)benzamide)pyridin-4-yl)oxy)-6-(2-methoxyethoxy)-N-methyl-1H-indole-1-carboxamide or a pharmacologically acceptable salt thereof; and
a preparation comprising an estrogen receptor antagonist or an aromatase inhibitor.

**16.** A method for treating breast cancer, comprising:
administering 5-((2-(4-(1-(2-hydroxyethyl)piperidin-4-yl)benzamide)pyridin-4-yl)oxy)-6-(2-methoxyethoxy)-N-methyl-1H-indole-1-carboxamide or a pharmacologically acceptable salt thereof; and an estrogen receptor antagonist or an aromatase inhibitor to a patient in need thereof.

**17.** 5-((2-(4-(1-(2-Hydroxyethyl)piperidin-4-yl)benzamide)pyridin-4-yl)oxy)-6-(2-methoxyethoxy)-N-methyl-1H-indole-1-carboxamideor a pharmacologically acceptable salt thereof for use in treatment of breast cancer, which is administered in combination with an estrogen receptor antagonist or an aromatase inhibitor.

**18.** A combination for treating breast cancer, comprising:

5-((2-(4-(1-(2-hydroxyethyl)piperidin-4-yl)benzamide)pyridin-4-yl)oxy)-6-(2-methoxyethoxy)-N-methyl-1H-indole-1-carboxamideor a pharmacologically acceptable salt thereof; and
an estrogen receptor antagonist or an aromatase inhibitor.

**19.** Use of 5-((2-(4-(1-(2-hydroxyethyl)piperidin-4-yl)benzamide)pyridin-4-yl)oxy)-6-(2-methoxyethoxy)-N-methyl-1H-indole-1-carboxamide or a pharmacologically acceptable salt thereof, which is administered in combination with an estrogen receptor antagonist or an aromatase inhibitor, for the manufacture of a therapeutic agent for breast cancer.

*Fig.1*

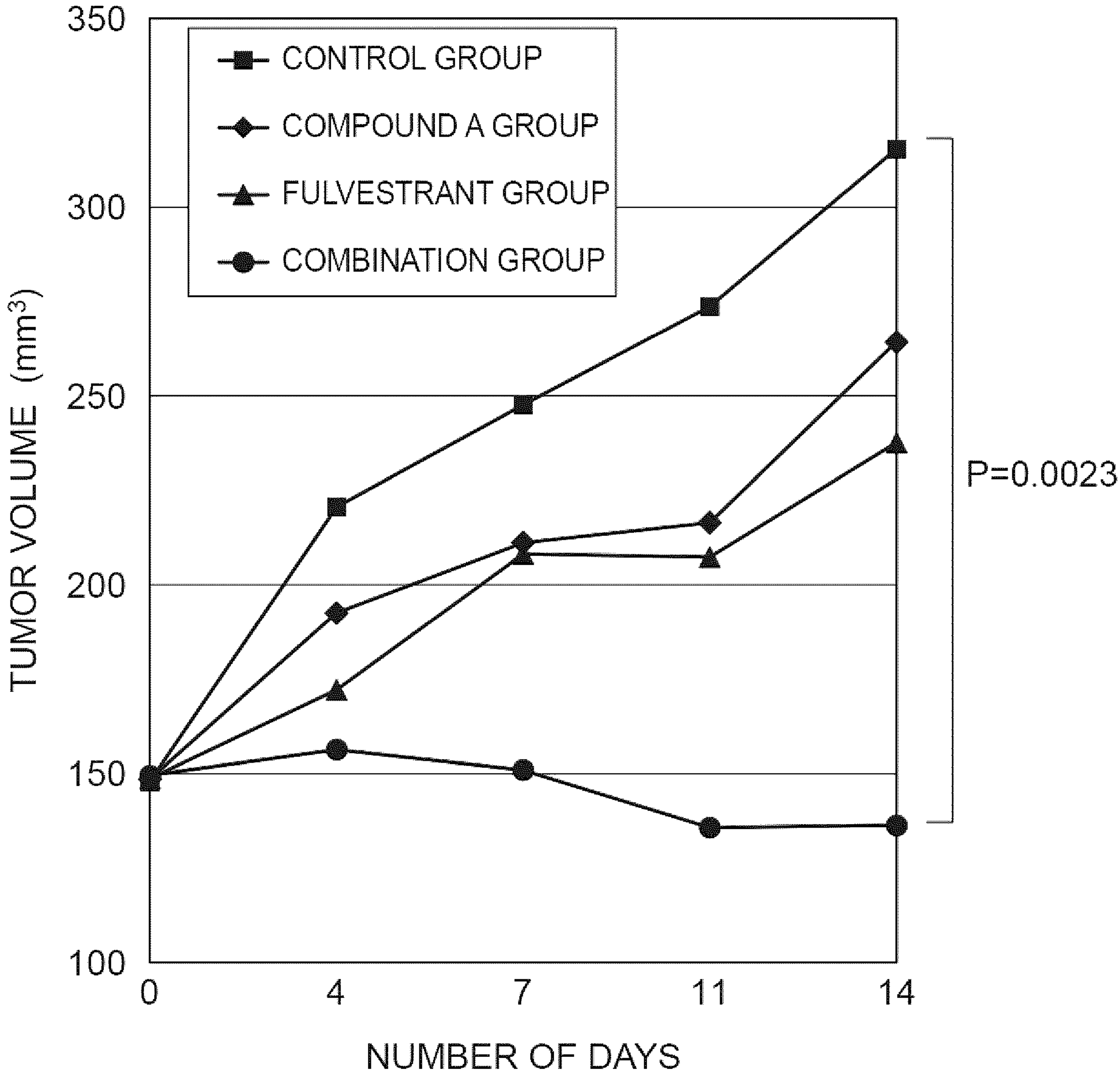
350
300
250
200
150
100
0
4
7
11
14
NUMBER OF DAYS
TUMOR VOLUME (mm3)
CONTROL GROUP
COMPOUND A GROUP
FULVESTRANT GROUP
COMBINATION GROUP
P=0.0023

*Fig.2*

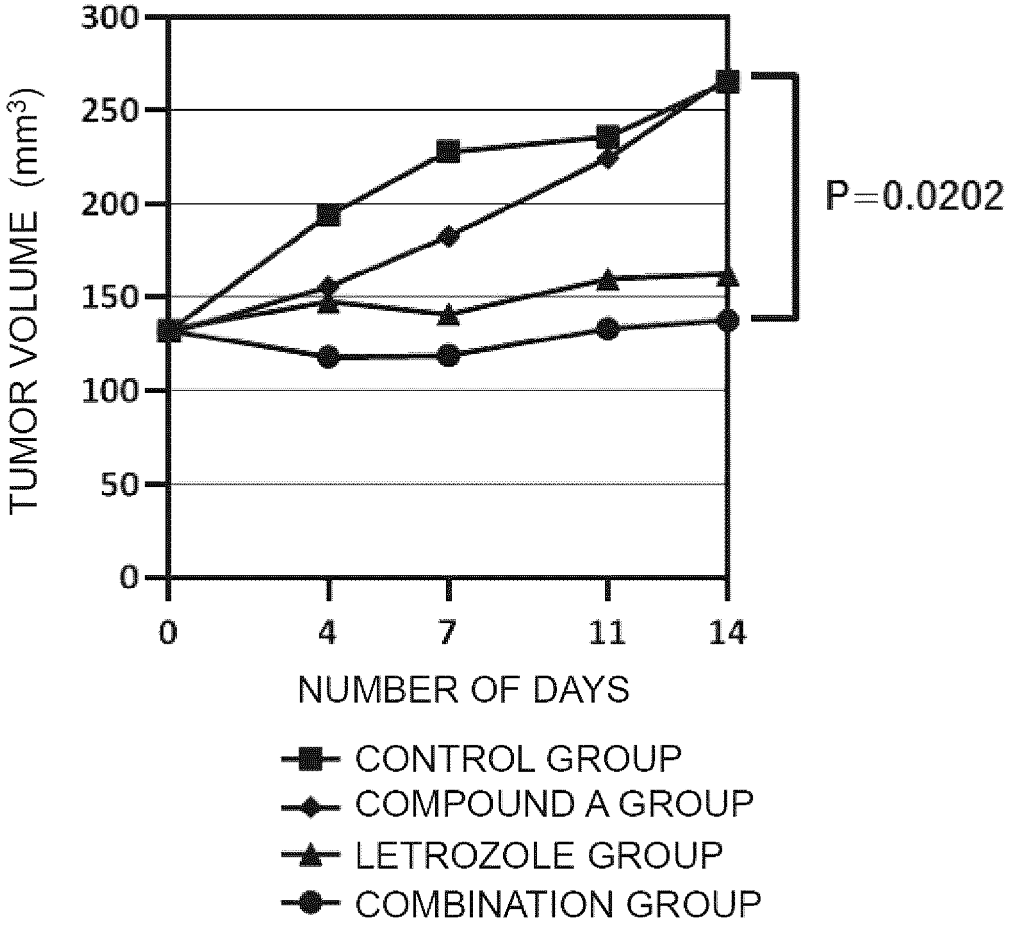
300
250
200
150
100
50
0
TUMOR VOLUME (mm3)
0
4
7
11
14
NUMBER OF DAYS
P=0.0202
CONTROL GROUP
COMPOUND A GROUP
LETROZOLE GROUP
COMBINATION GROUP

**INTERNATIONAL SEARCH REPORT**

International application No. PCT/JP2021/028008

A. CLASSIFICATION OF SUBJECT MATTER
Int.Cl. A61K31/4545(2006.01)i, A61K31/138(2006.01)i, A61K31/4196(2006.01)i, A61K31/565(2006.01)i, A61K31/5685(2006.01)i, A61K31/58(2006.01)i, A61K45/00(2006.01)i, A61P15/00(2006.01)i, A61P35/00(2006.01)i, A61P35/04(2006.01)i, A61P43/00(2006.01)i
FI: A61K31/4545, A61K31/565, A61K31/58, A61K31/5685, A61K31/4196, A61K31/138, A61K45/00, A61P35/00, A61P15/00, A61P43/00111, A61P43/00121, A61P35/04
According to International Patent Classification (IPC) or to both national classification and IPC

B. FIELDS SEARCHED

Minimum documentation searched (classification system followed by classification symbols)
Int.Cl. A61K31/4545, A61K31/138, A61K31/4196, A61K31/565, A61K31/5685, A61K31/58, A61K45/00, A61P15/00, A61P35/00, A61P35/04, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | WO 2017/104739 A1 (EISAI R & D MANAGEMENT CO., LTD.) 22 June 2017 (2017-06-22), claims, production example 1 | 17 |
| Y | claims, paragraphs [0002], [0003], production example 1, example 1 | 1-16, 18, 19 |
| X | MIYANO, WATANABE, Saori et al., E7090, a novel selective inhibitor of fibroblast growth factor receptors, displays potent antitumor activity and prolongs survival in preclinical models, Molecular Cancer Therapeutics, 2016, vol. 15, no. 11, pp. 2630-2639, ISSN 1535-7163, fig. 1 | 17 |
| Y | pp. 2631, 2634, 2635, table 2 | 1-16, 18, 19 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>23 August 2021 | Date of mailing of the international search report<br>31 August 2021 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/028008

C (Continuation). DOCUMENTS CONSIDERED TO BE RELEVANT

| Category* | Citation of document, with indication, where appropriate, of the relevant passages | Relevant to claim No. |
|---|---|---|
| X | KATOH, Masaru, Fibroblast growth factor receptors as treatment targets in clinical oncology, Nature Reviews Clinical Oncology, 2019, vol. 16, no. 2, pp. 105-122, ISSN 1759-4774, table 1 | 17 |
| Y | p. 117, supplementary table 1 | 1-16, 18, 19 |
| Y | MUSOLINO, A. et al., Phase II, randomized, placebo-controlled study of dovitinib in combination with fulvestrant in postmenopausal patients with HR+, HER2- breast cancer that had progressed during or after prior endocrine therapy, Breast Cancer Research, 2017, vol. 19, p. 18, ISSN 1465-542X, abstract, background, discussion | 1-16, 18, 19 |
| Y | FORMISANO, L. et al., Association of FGFR1 with ERα maintains ligand-independent ER transcription and mediates resistance to estrogen deprivation in ER+ breast cancer, Clinical Cancer Research, 2017, vol. 23, no. 20, pp. 6138-6150, ISSN 1078-0432, abstract, pp. 6148, 6149 | 1-16, 18, 19 |
| Y | JP 2015-505562 A (NOVARTIS AG) 23 February 2015 (2015-02-23), claims, paragraphs [0015]-[0018], [0023]-[0025], examples | 1-16, 18, 19 |
| Y | QUINTELA-FANDINO, M. et al., Nintedanib plus letrozole in early breast cancer: a phase 0/I pharmacodynamic, pharmacokinetic, and safety clinical trial of combined FGFR1 and aromatase inhibition, Breast Cancer Research, 2019, vol. 21, p. 69, ISSN 1465-542X, abstract, background, discussion, conclusion | 1-16, 18, 19 |
| Y | SECKL, M. et al., RADICAL trial: A phase Ib/IIa study to assess the safety and efficacy of AZD4547 in combination with either anastrozole or letrozole in ER positive breast cancer patients progressing on these aromatase inhibitors (AIs), Journal of Clinical Oncology, vol. 35, no. 15, supplement 1, 2017, (abstract) EMBASE [online], [retrieved on 23 August 2021], retrieved from: STN, AN 0052652985, abstract | 1-16, 18, 19 |

Form PCT/ISA/210 (second sheet) (January 2015)

**INTERNATIONAL SEARCH REPORT**
Information on patent family members

International application No.
PCT/JP2021/028008

| | | |
|---|---|---|
| WO 2017/104739 A1 | 22 June 2017 | EP 3391885 A1<br>claims, paragraphs [0002], [0003], production example 1, example 1<br>US 2018/0303817 A1<br>CA 3001969 A<br>AU 2016374441 A<br>CN 108367000 A<br>KR 10-2018-0094862 A<br>MX 2018006329 A<br>BR 112018010103 A<br>RU 2018119102 A |
| JP 2015-505562 A | 23 February 2015 | WO 2013/116293 A1<br>claims, pages 4, 5, 41-45<br>US 2014/0378422 A1<br>EP 2809312 A1<br>AU 2013215251 A<br>CA 2861377 A<br>CN 104093402 A<br>KR 10-2014-0117457 A<br>MX 2014009303 A<br>RU 2014135436 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140235614 A
- US 20180015079 A
- US 20180303817 A
- WO 2019189241 A
- US 20170217935 A


### Non-patent literature cited in the description


- **HOWELL et al.** Comparison of Fulvestrant Versus Tamoxifen for the Treatment of Advanced Breast Cancer in Postmenopausal Women Previously Untreated With Endocrine Therapy: A Multinational, Double-Blind, Randomized Trial. *Journal of Clinical Oncology,* 2004, vol. 22 (9), 1605-1613
- **DEEKS et al.** Exemestane A Review of its Use in Postmenopausal Women with Breast Cancer. *Drugs,* 2009, vol. 69 (7), 889-918